# EUROPEAN PATENT APPLICATION

(11) **EP 1 844 770 A1**
(43) Date of publication of application: **17.10.2007**
(21) Application number: 06712600.3
(22) Date of filing: 30.01.2006
(51) Int. Cl.: A61K 31/198, A61P 1/16, A61K 9/20, A61K 47/26, A61K 47/36, A61K 47/38, A61K 47/40, A61P 3/04, A61P 5/10

(54) **TABLETS CONTAINING ORNITHINE HYDROCHLORIDE**

(30) Priority: 31.01.2005 JP 2005022851
(71) Applicant: KYOWA HAKKO KOGYO CO., LTD., Chiyoda-ku, Tokyo 100-8185 (JP)
(72) Inventor: KAMIYA, Toshikazu c/o Healthcare Products Development Center,, Tsukuba-shi, Ibaraki 305-0841 (JP); KIMURA, Masao c/o Healthcare Products Development Center,, Tsukuba-shi, Ibaraki 305-0841 (JP); SAKAI, Yasushi c/o Healthcare Products Development Center,, Tsukuba-shi, Ibaraki 305-0841 (JP)
(74) Representative: Dossmann, Gérard
(86) International application number: PCT/JP2006/301457
(87) International publication number: WO 2006/080498

(57) **Abstract**

The present invention has an object to provide a tablet capable of comprising ornithine hydrochloride in a higher content; and a process for producing the same, and provides the tablet comprising ornithine hydrochloride and maltose, or a linear or cyclic dextrin; and the process for producing a tablet comprising ornithine hydrochloride, which comprises compression molding a mixture comprising ornithine hydrochloride and maltose, or a linear or cyclic dextrin by a direct tableting method.

## Description

### Technical Field

The present invention relates to a tablet comprising ornithine hydrochloride and a process for producing the same.

### Background Art

L-ornithine is known to stimulate the pituitary gland thereby to secrete a growth hormone ("Clinical Endocrinology", vol. 17, No. 2, pp. 119-122, 1982), to promote the secretion of a growth hormone thereby to promote protein synthesis and enhance muscle synthesis ("Annals of Surgery", vol. 206, No. 5, pp. 674-678, 1987), and to improve hyperammonemia caused by disorder of liver function thereby to prevent hepatic encephalopathy ("Journal of Pharmacology & Experimental Therapeutics", vol. 283, No. 1, pp. 1-6, 1997). Pharmaceutical preparations comprising L-ornithine as an active ingredient are also known as supplement products intended to prevent obesity and to promote skin restoration, and pharmaceutical products for liver damage ("Amino Acids", vol. 3, pp. 147-153, 1992).

Ornithine hydrochloride is widely used as an inexpensive and stable raw material for L-ornithine. As a tablet comprising ornithine hydrochloride, a tablet comprising L-ornithine hydrochloride in an amount of 43.5% by mass in the tablet ("Remake Ornithine" manufactured by Kyowa Hakko Kogyo Co., Ltd.) is available in the market.

On the other hand, the use of a cyclodextrin as an additive in the production of a tablet has been known. For example, a direct tableting aid comprising a tablet excipient such as microcrystalline cellulose and a binder such as β-cyclodextrin (see Patent document 1); a granule obtained by spray-coating saccharides, examples of which include a monosaccharide, a disaccharide and a sugar alcohol, with a solution in which a cyclodextrin is completely dissolved and granulating the resulting material, and a compressed tablet derived from the granulate (see Patent document 2) and the like have been disclosed.
Patent document 1: JP-A-1993-339197
Patent document 2: JP-A-2002-255796

### Disclosure of the Invention

### Problems to be Solved by the Invention

An object of the present invention is to provide a tablet comprising ornithine hydrochloride in a higher content and a process for producing the same.

### Means for Solving the Problems

The present invention relates to the following (1) to (9).
(1) A tablet comprising ornithine hydrochloride and maltose, or a linear or cyclic dextrin.
(2) The tablet according to the above (1), wherein the content of ornithine hydrochloride is 50% or more by mass in the tablet.
(3) The tablet according to the above (1) or (2), wherein the content of the maltose, or the linear or cyclic dextrin is from 5 to 20% by mass in the tablet.
(4) The tablet according to any one of the above (1) to (3), wherein the maltose, or the linear or cyclic dextrin is β-cyclodextrin.
(5) The tablet according to any one of the above (1) to (4), further comprising cellulose or a cellulose derivative.
(6) The tablet according to the above (5), wherein the cellulose or the cellulose derivative is microcrystalline cellulose with a particle size of from 20 to 60 µm.
(7) The tablet according to any one of the above (1) to (6), wherein the tablet hardness is from 30 to 150 N.
(8) A process for producing a tablet comprising ornithine hydrochloride, characterized by compression molding a mixture comprising ornithine hydrochloride and maltose, or a linear or cyclic dextrin by a direct tableting method.
(9) A process for producing a tablet comprising ornithine hydrochloride, characterized by comprising the steps of: granulating a mixture comprising ornithine hydrochloride and maltose, or a linear or cyclic dextrin; and performing compression molding the granulated mixture.

### Effect of the Invention

According to the present invention, a tablet comprising ornithine hydrochloride in a higher content and a process for producing the same can be provided.

### Best Mode for Carrying Out the Invention

The tablet of the present invention contains ornithine hydrochloride and maltose, or a linear or cyclic dextrin, and preferably further contains cellulose or a cellulose derivative.

The tablet of the present invention may contain a saccharide, a lubricant, a glidant, a sweetener, an acid, a binder, an antioxidant, a coloring agent, a flavor, a disintegrant and the like as needed.

The ornithine hydrochloride in the present invention may be any of L-form, D-form and a mixture of L- and D-forms, and preferred examples thereof include L-ornithine hydrochloride ((S)-diaminopentanoic acid monohydrochloride). The ornithine hydrochloride in the present invention is preferably a crystal or a crystalline powder, the purity thereof is preferably 98% or higher, and the loss of drying is preferably 0.2% or less. A process for producing the ornithine hydrochloride is not particularly limited, and it can be produced by a production process including a fermentation method, a synthetic method, a purification method and the like. The particle size distribution of ornithine hydrochloride is not particularly limited. The ornithine hydrochloride can be purchased from, for example, Kyowa Hakko Kogyo Co. , Ltd. , Kyowa Wellness Co. , Ltd. or the like (product name: L-ornithine hydrochloride). In the tablet of the present invention, it is possible to incorporate ornithine hydrochloride in an amount of 50% by mass or more in the tablet. It is also possible to incorporate it in an amount of 60% by mass or more therein.

The maltose, or the linear or cyclic dextrin in the present invention refer to a saccharide having a skeleton in which two to several tens of pyranose-type glucose molecules are linked linearly or circularly through an α-1,4 linkage. Further, a branched chain consisting of 1 to 3 glucose molecules, or an ether formed by the substitution of hydrogen of at least one hydroxyl group on the skeleton with C₁₋₆ alkyl, hydroxylated C₁₋₆ alkyl, carboxylated C₁₋₆ alkyl, or C₁₋₆ alkyloxycarbonylated C₁₋₆ alkyl may be contained in the skeleton in which the molecules are linearly or circularly linked. The maltose, or the linear or cyclic dextrin may be either a compound naturally occurring or a compound obtained by a chemical synthetic method. Further, a commercially available compound can also be used, and either a purified product or a partially purified product can be used.

Examples of the linear or cyclic dextrin in the present invention include maltodextrin, dextrose, α-cyclodextrin, β-cyclodextrin, γ-cyclodextrin and the like, and preferred examples thereof include α-cyclodextrin, β-cyclodextrin and γ-cyclodextrin, and more preferred examples thereof include β-cyclodextrin. The average particle size of the maltose, or the linear or cyclic dextrin is, for example, from 1 to 150 µm, preferably from 5 to 100 µm, more preferably from 10 to 80 µm, and particularly preferably from 30 to 60 µm. The average particle size can be measured using, for example, a laser diffraction particle size distribution analyzer (HEROS & RODOS, manufactured by JEOL Ltd.).

The water content in the maltose, or the linear or cyclic dextrin is preferably from 0 to 10% by mass, and more preferably from 0.01 to 5% by mass.

The above-mentioned values of the average particle size and water content of the maltose, or the linear or cyclic dextrin in the present invention are preferred also from the point of view of an action of further preventing a decrease in the quality of the tablet due to being browned. Further, for example, in the case where the cyclic dextrin is used among the maltose, the linear dextrin and cyclic dextrin, the resulting tablet is more unlikely to be browned, therefore, it is more preferred to select the cyclic dextrin from the point of view associated with the quality of the tablet.

The compounding ratio of the maltose, or the linear or cyclic dextrin in the tablet of the present invention is preferably from 1 to 50% by mass, and more preferably from 5 to 20% by mass.

Examples of the cellulose or cellulose derivative in the present invention include microcrystalline cellulose, powdered cellulose, calcium carboxymethyl cellulose and the like, and preferred examples thereof include microcrystalline cellulose and the like. The particle size of the cellulose or cellulose derivative is not particularly limited, however, it is preferably from about 20 to 60 µm. The particle of the cellulose or cellulose derivative is preferably a particle with a bulk density of from 0.15 to 0.4 g/cm³ in order to further increase the hardness, disintegratability or the like of the tablet, and further it is preferably a particle with an angle of repose of from 40 to 50° in order to further increase the content uniformity, production rate or the like of the tablet. A process for producing the cellulose or cellulose derivative is not particularly limited, however, the microcrystalline cellulose can be produced by, for example, hydrolyzing pulp fiber or the like, and removing non-microcrystalline cellulose, and then pulverizing and drying the resulting residue.

The compounding ratio of the cellulose or cellulose derivative in the tablet of the present invention is not particularly limited as long as the amount thereof is in the range generally used in a pharmaceutical preparation. However, it is preferably from.1 to 30% by mass, and more preferably from 10 to 25% by mass.

The saccharide is not particularly limited as long as it can be used in a food or the like, however, examples thereof include monosaccharides, disaccharides, sugar alcohols, oligosaccharides and the like, and preferred examples thereof include sugar alcohols.

Examples of the monosaccharide include glucose, xylose, galactose, fructose and the like. Examples of the disaccharide include trehalose, sucrose, lactose, paratinose and the like. Examples of the sugar alcohol include maltitol, erythritol, sorbitol, xylitol and the like. Examples of the oligosaccharide include raffinose, inulooligosaccharide (an oligosaccharide derived from chicory), palatinose oligosaccharide and the like.

The form of the saccharide is not particularly limited, however, it is preferably in the form of a microcrystal or a microparticle. The average particle size thereof is, for example, from 1 to 100 µm, preferably from 5 to 80 µm, more preferably from 10 to 60 µm, and particularly preferably from 30 to 50 µm. The compounding ratio of the saccharide in the tablet of the present invention is not particularly limited as long as the amount thereof is in the range generally used in a pharmaceutical preparation.

The lubricant is not particularly limited as long as it can be used in a food or the like, however, examples thereof include stearic acid or metal salts thereof such as stearic acid, magnesium stearate and calcium stearate, sucrose fatty acid esters or glycerol fatty acid esters, hydrogenated oils and fats, silicon dioxide, calcium phosphate and the like. The lubricant may be present only on the surface of the tablet or may be dispersed inside the tablet. The compounding ratio of the lubricant in the tablet of the present invention is preferably from 0.01 to 20% by mass and more preferably from 0.3 to 5% by mass.

The glidant is not particularly limited as long as it can be used in a food or the like, however, examples thereof include calcium phosphate, calcium hydrogen phosphate, microparticulate silicon dioxide and the like. The compounding ratio of the glidant in the tablet of the present invention is preferably from 0 to 20% by mass, more preferably from 0.01 to 10% by mass and particularly preferably from 0.05 to 5% by mass.

The sweetener is not particularly limited as long as it can be used in a food or the like, however, examples thereof include sodium saccharin, dipotassium glycyrrhizinate, aspartame, stevia, thaumatin and the like. The compounding ratio of the sweetener in the tablet of the present invention is not particularly limited as long as the amount thereof is in the range generally used in a pharmaceutical preparation.

The acid is not particularly limited as long as it can be used in a food or the like, however, examples thereof include citric acid, tartaric acid, malic acid and the like. The compounding ratio of the acid in the tablet of the present invention is not particularly limited as long as the amount thereof is in the range generally used in a pharmaceutical preparation.

The binder is not particularly limited as long as it can be used in a food or the like, however, examples thereof include gelatin, pullulan and the like. The compounding ratio of the binder in the tablet of the present invention is not particularly limited as long as the amount thereof is in the range generally used in a pharmaceutical preparation.

The antioxidant is not particularly limited as long as it can be used in a food or the like, however, examples thereof include tocopherol, ascorbic acid, cysteine hydrochloride, L-ascorbate stearate esters and the like. The compounding ratio of the antioxidant in the tablet of the present invention is not particularly limited as long as the amount thereof is in the range generally used in a pharmaceutical preparation.

The coloring agent is not particularly limited as long as it can be used in a food or the like, however, examples thereof include Yellow #5 dye for food, Red #2 dye for food, Blue #2 dye for food, carotenoid pigments, tomato pigments and the like. The compounding ratio of the coloring agent in the tablet of the present invention is not particularly limited as long as the amount thereof is in the range generally used in a pharmaceutical preparation.

The flavor is not particularly limited as long as it can be used in a food or the like, however, examples thereof include lemon flavor, lemon lime flavor, grapefruit flavor, apple flavor and the like. The compounding ratio of the flavor in the tablet of the present invention is not particularly limited as long as the amount thereof is in the range generally used in a pharmaceutical preparation.

The disintegrant is not particularly limited as long as it can be used in a food or the like, however, examples thereof include cornstarch, potato starch and the like. The compounding ratio of the disintegrant in the tablet of the present invention is not particularly limited as long as the amount thereof is in the range generally used in a pharmaceutical preparation.

The tablet of the present invention preferably has a hardness such that, for example, the tablet does not have a chip or does not crumble. The tablet hardness is measured by generally using a tablet hardness tester as a breaking strength in the diametrical direction of the tablet, and the value is preferably from 20 to 200 N, more preferably from 30 to 150 N, particularly preferably from 40 to 100 N. The tablet hardness can be measured using a commercially available measuring device of tablet breaking strength such as TH-203CP manufactured by Toyama Sangyo Co., Ltd.

The shape of the tablet of the present invention is not particularly limited, however, for example, a round tablet, a triangular tablet and a cannonball tablet and the like are preferred. The size of the tablet of the present invention is not particularly limited, however, it is preferred that the mass thereof is from 0.1 to 2 g and the diameter thereof is from 0.3 to 2.0 cm.

The tablet of the present invention can be produced by, for example, a production process including the steps of: mixing the respective above-mentioned constituent components of the tablet in a state of a powder as such or granulating a part of the constituent components followed by mixing it with the rest of the constituent components, or granulating all the constituent components; and subsequently compression molding the resulting mixture or granule thereby to produce a tablet. More specifically, a tablet with a desired hardness can be produced even by a production process in which the constituent components of the tablet comprising ornithine hydrochloride and maltose, or a linear or cyclic dextrin, preferably further comprising cellulose or a cellulose derivative are mixed, and the resulting mixture is compression molded by a direct tableting method. In addition, a tablet with a desired hardness can be produced even by a production process in which a mixture comprising at least ornithine hydrochloride and maltose, or a linear or cyclic dextrin among the respective constituent components of the tablet comprising ornithine hydrochloride and maltose, or a linear or cyclic dextrin, preferably further comprising cellulose or a cellulose derivative is granulated, followed by mixing it with the rest of the constituent components and subsequently compression molding the resulting mixture (a production process in which compression molding is carried out by an indirect tableting method). The apparatus to be used for the compression molding is not particularly limited, and a compression machine such as a rotary compression molding machine or a hydraulic press machine can be used. The above-mentioned production process in which compression molding is carried out by a direct tableting method is an extremely convenient production process because the respective constituent components of the tablet are only mixed and subjected to compression molding, which is therefore preferred. Further, because water or the like is not added to the constituent components during the production process, it is preferred also from the point of view of the stability of the constituent components of the tablet and the like.

The tablet of the present invention can also be produced by a so-called externally lubricating compression molding method, in which an extremely small amount of a lubricant is applied on a punch and a die of a compression molding machine in advance and a mixture which does not contain a lubricant is compression molded using the compression molding machine having the punch and die on which the lubricant has been applied.

Examples of the granulation method in the case of granulating a part of or all the constituent components of the tablet of the present invention (for example, the above-mentioned production process in which compression molding is carried out by an indirect tableting method or the like) include a wet-granulation method using purified water, ethanol or the like, a dry-granulation method and the like. The apparatus to be used for granulation is not particularly limited, and for example, a fluidized bed granulator, a tumbling agitation granulator, an extrusion granulator or the like can be used. The production process in which compression molding is carried out by an indirect tableting method is preferred because the contents of ornithine hydrochloride (50% or more by mass in the tablet), maltose, or a linear or cyclic dextrin, and cellulose or a cellulose derivative can be made uniform. Further, it is preferred to reduce the addition of water or the like during granulation as much as possible from the point of view of the stability of the constituent components of the tablet and the like.

The tablet of the present invention may be a sugar-coated tablet coated with, for example, a saccharide, a sugar alcohol or the like or a coated tablet so as to improve a moisture-proof property, storage stability or the like.

The tablet of the present invention can be taken as a food, a pharmaceutical product or the like, and in the case of taking it in the form of a tablet food for the purpose of daily nutrition intake, the intake amount in terms of ornithine hydrochloride preferably is from 100 mg to 2 g. With the use of the tablet of the present invention, it is possible to reduce the number of tablets to be taken by incorporating ornithine hydrochloride in an amount of 50% by mass or more in the tablet. Preferably, ornithine hydrochloride is incorporated in the tablet in an amount of 60% by mass or more thereby to enable the further reduction of the number of tablets to be taken. It is preferred that the number of tablets to be taken per day is set in the range of from 1 to 15.

Hereinafter, the present invention will be described in further detail with reference to Examples, however, the present invention is not limited to these Examples.

### Example 1

After 68.1 g of L-ornithine hydrochloride (product name: L-ornithine hydrochloride (manufactured by Kyowa Hakko Kogyo Co., Ltd.)), 18.0 g of microcrystalline cellulose (product name: Avicel FD101 (manufactured by Asahi Kasei Chemicals Corporation), hereinafter the same shall apply), 3.3 g of a sucrose fatty acid ester (product name: DK ester F-20W, (manufactured by Daiichi Kogyo Seiyaku Co. , Ltd.), hereinafter the same shall apply), 0.6 g of calcium phosphate (product name: Tricalcium phosphate, (manufactured by Taihei Chemical Industrial Co., Ltd.), hereinafter the same shall apply) and 10.0 g of β-cyclodextrin (product name: Celdex B-100, (manufactured by Nihon Shokuhin Kako Co., Ltd.), hereinafter the same shall apply) were well mixed in a polyethylene bag, the resulting mixture was compression molded using a single compression molding machine (a vertical tableting machine 6B-2M, (manufactured by Kikusui Seisakusho Ltd.)) such that the tablet hardness (a measurement apparatus: a hardness tester KHT-20N (manufactured by Fujiwara Scientific Co., Ltd.), hereinafter the same shall apply) became 80 N, whereby a tablet of 250 mg with a diameter of 8 mm was obtained.
The tablet could be produced without causing any tableting problems. Further, even when the tablet was stored at 40°C for 1 month, a change in the appearance and properties was not observed.

### Example 2

A tablet was obtained in the same manner as in Example 1, except that 10.0 g of β-cyclodextrin in Example 1 was changed to 10.0 g of maltose (product name: Sunmalt Midori, (manufactured by Hayashibara Shoji Inc.).
The tablet could be produced without causing any tableting problems.

### Comparative example 1

Compression molding was carried out in the same manner as in Example 1, except that 10.0 g of β-cyclodextrin in Example 1 was not used, the weight of the microcrystalline cellulose was changed from 18.0 g to 28.4 g, the weight of the sucrose fatty acid ester was changed from 3.3 g to 3.0 g, and the weight of the calcium phosphate was changed from 0.6 g to 0.5 g.
Lamination was caused during molding, and a tablet could not be produced.

### Comparative examples 2 to 4

Compression molding was carried out in the same manner as in Example 1, except that 10.0 g of β-cyclodextrin in Example 1 was changed to 10.0 g of lactose (product name: TABLETTOSE 80 (manufactured by MEGGLE)), 10.0 g of D-mannitol (product name: D-mannitol (for oral administration) (manufactured by Nikken Fine Chemical Co., Ltd.)) and 10.0 g of partially pregelatinized starch (product name: PCS FC-30 (manufactured by Asahi Kasei Chemicals Corporation)), respectively.
Lamination was caused during molding in any cases, and a tablet with a sufficient hardness could not be obtained.

**[Table 1]**

| | Example 1 | Example 2 | Comparative example 1 | Comparative example 2 | Comparative example 3 | Comparative example 4 |
|---|---|---|---|---|---|---|
| Ornithine hydrochloride | 68.1 | 68.1 | 68.1 | 68.1 | 68.1 | 68.1 |
| Microcrystalline cellulose | 18.0 | 18.0 | 28.4 | 18.0 | 18.0 | 18.0 |
| β-cyclodextrin | 10.0 | - | - | - | - | - |
| Maltose | - | 10.0 | - | - | - | - |
| Lactose | - | - | - | 10.0 | - | - |
| D-mannitol | - | - | - | - | 10.0 | - |
| Partially pregelatinized starch | - | - | - | - | - | 10.0 |
| Sucrose fatty acid ester | 3.3 | 3.3 | 3.0 | 3.3 | 3.3 | 3.3 |
| Calcium phosphate | 0.6 | 0.6 | 0.5 | 0.6 | 0.6 | 0.6 |
| Tableting problems | Tableting was possible without causing any problems. | Tableting was possible without causing any problems. | Lamination was caused. | Lamination was caused. | Lamination was caused. | Lamination was caused. |

### Example 3

### Production example of tablet using continuous tableting machine (direct tableting)

136.2 kg of ornithine hydrochloride, 36.0 kg of microcrystalline cellulose, 6.6 kg of a sucrose fatty acid ester, 1.2 kg of calcium phosphate and 20.0 kg of β-cyclodextrin were mixed using a conical blender (CB-1200 Blender, (manufactured by Nihon Kansoki Co. , Ltd.), hereinafter the same shall apply). The resulting mixture was compression molded using a rotary compression molding machine (VIRGO 524 SS1AY, (manufactured by Kikusui Seisakusyo Ltd.), hereinafter the same shall apply) at a compression molding pressure of 10 kN, whereby a tablet of 250 mg with a diameter of 8 mm was produced. Compression molding problems were not observed, and the tablet hardness was 81 N (an average value of n being 20).

### Example 4

Production example of tablet using continuous tableting machine (granulation)

A mixture of 81.7 kg of ornithine hydrochloride and 12.0 kg of β-cyclodextrin is granulated with a 5% aqueous solution obtained by dissolving 0.6 kg of pullulan (product name: Pullulan PI-20 (manufactured by Hayashibara Co., Ltd.)) in water using a fluidized bed granulator (FLO-120 (manufactured by Freund Corporation)). To the resulting granule (78.6 kg), 17.5 kg of microcrystalline cellulose, 3. 3 kg of a sucrose fatty acid ester, 0.6 kg of calcium phosphate are added and mixed using a conical blender. The resulting mixture is compression molded using a rotary compression molding machine at a compression molding pressure of 10 kN, whereby a tablet of 250 mg with a diameter of 8 mm is produced.

### Industrial Applicability

According to the present invention, a tablet comprising ornithine hydrochloride in a higher content; and a process for producing the same can be provided.

## Claims

1. A tablet comprising ornithine hydrochloride and maltose, or a linear or cyclic dextrin.

2. The tablet according to claim 1, wherein the content of ornithine hydrochloride is 50% or more by mass in the tablet.

3. The tablet according to claim 1 or 2, wherein the content of the maltose, or the linear or cyclic dextrin is from 5 to 20% by mass in the tablet.

4. The tablet according to any one of claims 1 to 3, wherein the maltose, or the linear or cyclic dextrin is β-cyclodextrin.

5. The tablet according to any one of claims 1 to 4, further comprising cellulose or a cellulose derivative.

6. The tablet according to claim 5, wherein the cellulose or the cellulose derivative is microcrystalline cellulose with a particle size of from 20 to 60 µm.

7. The tablet according to any one of claims 1 to 6, wherein the tablet hardness is from 30 to 150 N.

8. A process for producing a tablet comprising ornithine hydrochloride, which comprises compression molding a mixture comprising ornithine hydrochloride and maltose, or a linear or cyclic dextrin by a direct tableting method.

9. A process for producing a tablet comprising ornithine hydrochloride, which comprising the steps of: granulating a mixture comprising ornithine hydrochloride and maltose, or a linear or cyclic dextrin; and performing compression molding the granulated mixture.
